Europäisches Patentamt

European Patent Office

Office européen des brevets

(19)

(11) Publication number: **0 120 573**
**A2**

(12) **EUROPEAN PATENT APPLICATION**

(21) Application number: **84300885.5**

(22) Date of filing: **13.02.84**

(51) Int. Cl.³: **A 23 K 1/00**
**A 23 K 1/14, C 12 P 19/14**
**//A23L1/214, A23N17/00**

(30) Priority: **15.02.83 GB 8304091**

(43) Date of publication of application:
**03.10.84 Bulletin 84/40**

(84) Designated Contracting States:
**AT BE CH DE FR IT LI NL SE**

(71) Applicant: **UNILEVER NV**
**Burgemeester s'Jacobplein 1 P.O. Box 760**
**NL-3000 DK Rotterdam(NL)**

(72) Inventor: **Hoogstad, Bruin**
**Utrechtsestraatweg 1**
**Nieuwegein(NL)**

(72) Inventor: **Jansen, Hans Wilfrudus Maria**
**Prof. Gerbrandy Laan 11**
**Woerden(NL)**

(72) Inventor: **Sijmons, Antonius Hendrikus**
**Engel de Ruyter Straat 12**
**Breukelen(NL)**

(74) Representative: **Butler, David John et al,**
**Unilever PLC Patents Division P.O. Box 68 Unilever**
**House**
**London EC4P 4BQ(GB)**

(54) **Manufacture of feed material.**

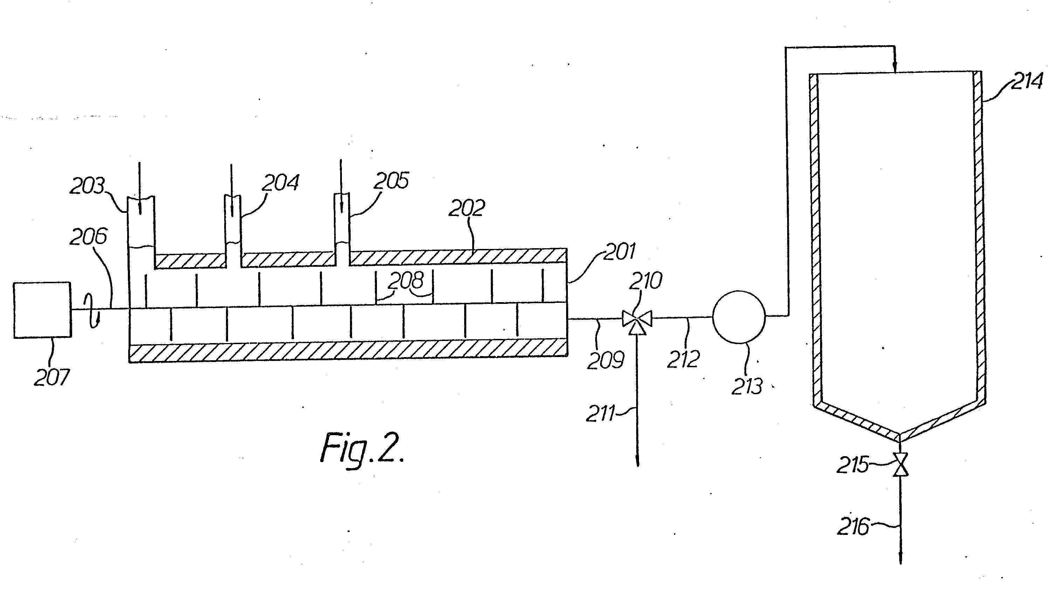

(57) A starch-rich meal, such as tapioca, is subjected to hydrolysis by a heat-stable alpha-amylase enzyme capable of hydrolysing starch starch to dextrins having a degree of polymerisation (DP) in the range 4 to 10, the hydrolysis being conducted at high solids content, typically greater than 60% by weight. This produces a viscous liquid product useful as a binder/conditioner in pelleted animal feedstuffs. The process is conducted preferably by passing the meal and enzyme rapidly through a heated reaction vessel (201 (Figure 2).

Fig.2.

EP 0 120 573 A2

## FEEDSTUFFS

The present invention relates to the manufacture of feedstuffs for animals, and particularly to the manufacture of partially-hydrolysed carbohydrate materials useful as ingredients in animal feedstuffs.

The invention provides a process for the manufacture of a starch-rich material useful as an animal feedstuff ingredient, in which process a starch-rich meal is subjected to hydrolysis by an alpha-amylase enzyme capable of hydrolysing starch to dextrins having a degree of polymerisation (DP) in the range 4 to 10, the hydrolysis being conducted at a total dry solids content of at least 50% by weight.

Preferably the starch-rich meal should contain at least 50% by weight starch, and ideally at least 60%. Starch rich meals from a wide variety of sources conventionally used in the manufacture of compound animal feeds can be used in the invention, e.g. potato starch, maize and wheat. However, a most preferred substrate is tapioca, i.e. meal from the root of the cassava (manioc) plant.

Preferably the meal should have a maximum particle size of less than 1000 microns.

Suitable enzymes can be obtained commercially from a variety of sources. In general these are bacterial in origin, and bacterial alpha-amylases are preferred. The enzyme should ideally break down starch into dextrins but not further into substantial quantities of monosaccharides or disaccharides. Moreover, ideally the enzyme should function rapidly at high temperature (preferably up to at least 100°C), so leading to maximum process efficiency. We have obtained good results using amylase enzyme that hydrolyses starch to dextrins having a degree of polymerisation (DP) of 5 glucose units. Depending on the completeness of the enzymatic reaction, much of the starch may remain in the form of longer molecules that will generally exhibit DP's in multiples of 5, for example 10 or 15. It will be appreciated that such values are not to be regarded as precise limits on the composition of the hydrolysed starch, as numerous other components will occur in at least trace amounts. The dominant components will exhibit the degree's of polymerisation stated. Other amylase enzymes are available that can hydrolyse starch to dextrins of other DP values, e.g. 6-7 (and of course multiples thereof), and these can be used as alternatives provided they are also capable of functioning rapidly at relatively high temperature. We believe that in general a "final" DP in the range 4 to 10 should be aimed for.

We have found that an ideal enzyme is available commercially from NOVO Industri A/S under the registered trade name TERMAMYL. In the manufacturer's literature this enzyme is described as being a heat-stable alpha-amylase, produced from a selected strain of Bacillus licheniformis, and further that it is an endo-amylase which will hydrolyse 1,4-alpha-glucosidic linkages in amylose and amylopectin at random, leading to dextrins of DP 5.

The process of the invention can be performed by blending enzyme with the starch-rich meal, in the presence of at least sufficient moisture to enable the enzymatic hydrolysis of starch to occur. Preferably the reaction mixture should be stirred or otherwise kept in a state of agitation throughout the hydrolysing process.

We believe that the moisture level in the reaction mixture will generally need to be at least 15% by weight, for a satisfactory enzymatic hydrolysis to take place. On the other hand, if the final product contains a high moisture level it may need to be subjected to severe or prolonged drying before it can be used as a feed ingredient, and this would be very wasteful in energy terms. This disadvantage has been a feature of processes described in the prior art. It is a distinct advantage of the process of the invention that it can be operated at quite low moisture levels and the product so formed can often be added directly to a compound feed formulation without any intervening drying. Typically the process of the invention can be operated at a moisture level of less than 50%, preferably less than 40%, and more preferably less than 30%, by weight. Thus, expressed in terms of truly dry solids content, the enzymatic hydrolysis of starch is preferably being conducted at solids contents of at least 60%, and indeed more preferably at least 70%, by weight. The use of such high solids contents in enzymatic reactions is unusual. The moisture in the reaction mixture can be derived from any combination of several sources. The starch-rich meal itself will normally contain some moisture, and hence "dry" meal is usually in equilibrium with its surroundings and may contain up to 20% by weight moisture. More usually, a moisture level of 10 to 16% by weight will be contributed by the meal itself. The enzyme will usually be added in aqueous slurry form, and indeed it is more easily handled in this form, although enzyme powders or granules can be used if desired. In addition, water can be added separately to the reaction mixture, and the direct injection of steam can thus be advantageous.

The reaction mixture should be heated to a temperature at which the enzyme can work efficiently. Amylase enzymes are available that can withstand temperatures of up to 100°C or more, and the operating temperature of a process of the invention is preferably in the range 80 to 110°C, and most preferably in the range 90-105°C. However, the process can be conducted at any temperature greater than, say, 60°C, provided that the particular enzyme used is not denatured. The manner in which such heating is achieved is not critical, but ideally it is conducted by means of indirect or direct steam heating. A combination of direct and indirect heating can be used if desired, and a very suitable batch-scale operation can be conducted by recycling the reaction mixture continuously through an indirect heat exchanger and simultaneously injecting steam into the reaction mixture.

Alternatively, as a continuous process, the meal and enzyme can be mixed and passed through a heated reaction chamber before being blended with other feed materials in a conventional compounding process.

An important feature of the invention is that the starch hydrolysis can be conducted very rapidly, especially in the context of a continuous process. We have found that processing times of less than 10 minutes, and indeed considerably less than 5 minutes, are quite adequate to achieve a reacted product that can be used as a feed ingredient.

Enzymes are generally sensitive to pH, and hence this parameter should be controlled in accordance with such advice as may be obtained from the enzyme supplier. However, in general, an aqueous slurry of starch-rich meal will have a pH well within the tolerance of any commercially-available enzyme. Generally a pH in the range 5 to 7 will be ideal.

A particularly important embodiment of the invention is a process in which a mixture of tapioca meal or the like, moisture and heat-stable endo-amylase enzyme capable of hydrolysing starch to a DP in the range of 4 to 10, at a total solids content in the mixture of at least 60% by weight, is heated to produce a highly-viscous feed ingredient. This feed ingredient can be used to particular advantage in pelleted animal feedstuffs, wherein it is found to increase substantially the hardness of such pelleted feedstuffs. The advantage of increased pellet hardness can be exploited as such, or it can be used to offset the negative physical effects of other useful processing/formulation changes. For example, increasing the throughput of traditional ring-roll pelleting presses can have an adverse effect on pellet hardness. Raising the level of fat/oil in the formulation generally leads to a softer pellet, and this can be offset

0120573

by means of the novel material of the invention. Similar physical benefits can be observed by including the material of the invention in feeds formed into other compressed particulate shapes, e.g. briquettes or blocks.

These physical benefits can be obtained by including just a minor amount of the partially- hydrolysed material of the invention in the feedstuff.  When the partially-hydrolysed starch material is used as a binder/conditioner in a pelleted feedstuff, it will generally comprises about 1-10% by weight of the formulation, although higher levels (up to 20% or more, by weight) can be added if desired.

The partially-hydrolysed starch material of the invention can be blended with conventional animal feedstuff ingredients to make a compound feed.  The physical preparation of the compound feed can be achieved using any conventional equipment.  The nature of the feedstuff ingredients that can be mixed with the partially-hydrolysed starch material is not critical to the invention, and these can be any nutritionally appropriate combination of the ingredients used in feed manufacture, e.g. grains, either whole or milled, such as barley, wheat, sorghum, maize, rice oats and rye; cereal by-products, such as wheat feed (germ), maize dark grains, barley dark grains, brewers grains, malt culms (sprouting tips from the brewing industry), maize germ, grain screenings and oat feed; oil seed residues derived from seeds such as rape, soya, groundnut, palm kernel, sun flower, linseed, shea nut, illipe and cotton-seed; oils and fats of vegetable or animal origin; any of the miscellaneous products of plant or animal origin conventionally used in feedstuff manufacture, such as field beans, peas, tapioca, beet pulp, potato pulp, straw, guar, molasses, single-cell protein, meat and bone meal, fish meal, blood meal and dried poultry manure;
non-protein nitrogen sources such as urea and urea
derivatives; and vitamins and mineral additives.

Alternatively, the partially-hydrolysed starch
material of the invention can be dried as necessary, for
example by drum-drying, belt-drying or spray-drying, to
yield a solid material which can be milled to a powder.
The solid material be be blended with other feed materials
in the dry state, or mixed with water or other edible
liquids if desired.  Unlike molasses, the product of the
invention can be dried to a solid which in particulate
form is free-flowing and relatively non-hygroscopic.

If desired, the partially-hydrolysed starch material
can be blended with a carrier material, such as milled
grain, and then dried.  For example, partially-hydrolysed
tapioca can be blended with untreated tapioca meal, e.g.
in a 1:1 ratio.

An important aspect of the invention is the use of
the partially-hydrolysed material as a conditioning agent
in non-hydrolysed starch-rich meal.  For example, the
partially-hydrolysed material can be incorporated as a
minor ingredient in non-hydrolysed material, and the
mixture then pelleted.  This is especially suitable as a
means for improving the handling properties of tapioca:
Partially hydrolysed tapioca produced in accordance with
the invention should be mixed with unmodified tapioca, and
pelleted.  The pelleted tapioca is less likely to break up
and cause dust problems during subsequent handling and
transport, and hence its quality as a feed ingredient is
maintained during shipment.  This benefit can be obtained
even when the inclusion level of the partially-hydrolysed
tapioca is very low, e.g. 1% or less of the pelleted
material.

By way of example only, two preferred embodiments of the invention will now be described with reference to the accompanying drawings, which depict the general layout of equipment that can be used in the partial hydrolysis of starch in accordance with the invention.

Figure 1

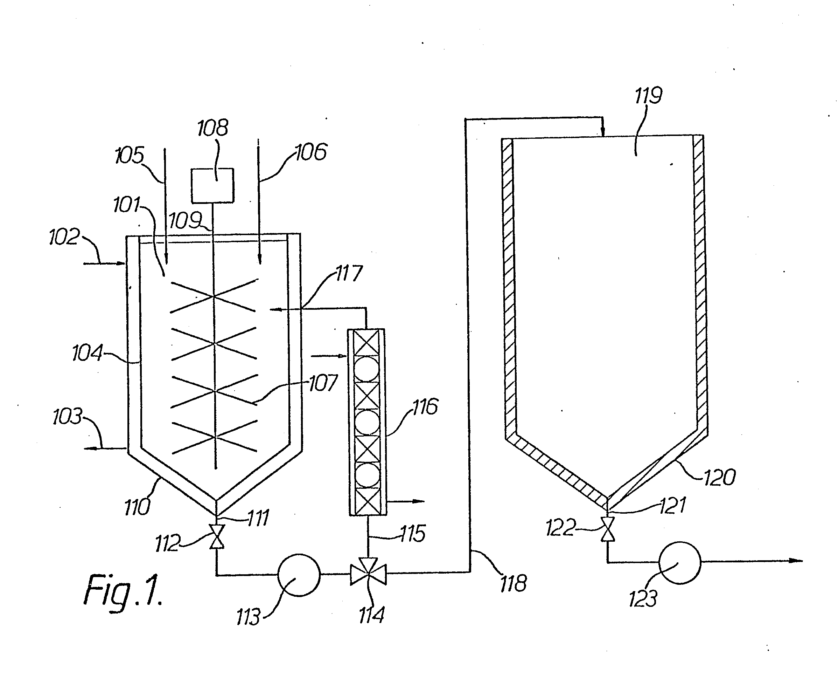

Referring to Figure 1, the apparatus of the first embodiment comprises a jacketed reaction vessel 101 with an inlet 102 and outlet 103 for admitting steam or hot water into the surrounding jacket 104. Reaction vessel 101 is provided with an inlet 105 for steam or water and an inlet 106 for other materials such as meal and enzyme. Stirring means 107 is connected to a drive means by a shaft 109. Lower end 110 of reaction chamber 101 is provided with an outlet pipe 111 leading via valve 112 and pump 113 to valve 114. From valve 114 a recirculation pipe 115 leads via a jacketed heat exchanger 116 to inlet 117 in reaction chamber 101. Alternative route 118 from valve 114 leads into lagged storage vessel 119 from the lower end 120 of which an outlet pipe 121 leads via valve 122 and pump 123 into a conventional feedstuff processing line (not shown).

In operation, starch-rich meal is fed into reaction vessel 101 via inlet 106 and blended as necessary with water or steam from inlet 105. Reaction vessel 101 is heated via heating jacket 104 and the contents of the reaction vessel are stirred and recirculated via heat exchanger 116 until appropriate temperature conditions are reached for enzyme reaction. At this stage, enzyme slurry is added via inlet 106 and the heating and recirculation of the contents of reaction vessel 101 continued until a desired degree of enzyme reaction has taken place. The contents of the reaction vessel can then be diverted via valve 114 into lagged storage vessel 119 from which the reaction product can be fed as required into the main processing line. In the apparatus as depicted in Figure 1 it is envisaged that the recirculation system will be used to prepare batches of enzyme-treated material which can be stored in bulk in he storage vessel until required. It will be appreciated however, that the apparatus depicted can be used to prepare enzyme-treated material in a more continuous operation, for example, by blending the ingredients in the reaction chamber and feeding the contents directly to the storage vessel in which the reaction can be allowed to proceed to completion.

Example 1

Equipment as just described with reference to Figure 1 was used to prepare batches of enzyme-treated tapioca meal as follows.

Initially the reaction vessel was partially filled with water at 100°C, and tapioca meal and enzymes slurry added progressively while the contents of the vessel were stirred and heated by steam, until a final solids content of 65% by weight was attained. The tapioca meal had been milled to a particle size of less than 300 microns, and contained its natural moisture content of about 12% by weight. The enzyme was a commercially-available alpha-amylase ("TERMAMYL 120L") in aqueous slurry form as obtained from the supplier, and was added at the level of 600 grams of slurry per 1000 kilos of meal. The mixture was recirculated at a temperature of about 100°C for approximately 20 minutes and the batch was then transferred to the holding tank.

The treated tapioca meal was used as a substitute for molasses in a conventional compound pelleted cattle feed at levels varying from about 4 to about 16% by weight of the finished product. For the purposes of comparison, a conventional cattle feed of identical formulation but containing instead molasses at the same inclusion levels was prepared. At all inclusion levels it was noted that the finished product containing the treated tapioca meal slurry of the invention was consistently harder (i.e. the pellets were less liable to break up) than in the molasses-containing conventional product.

It was also noted that after 48 hours the hardness of the product of the invention had substantially increased. A similar "curing" effect is generally noted with conventional feedstuffs containing molasses, but in this instance the degree of "curing" observed in the product of the invention was substantially greater than that in the comparable mollasses-containing product.

## Figure 2

Referring to Figure 2, the second embodiment of the invention comprises a horizontal cylindrical reaction vessel 201 having a thermal jacket 202, an inlet 203 for carbohydrate material, an inlet 204 downstream of inlet 203 for enzyme slurry, and a further inlet 205 downstream of inlet 204 for steam. Rotatable shaft 206 extends along the full axial length of reaction vessel 201 and can be rotated by motor means 207. A plurality of vanes 208 are arranged along shaft 206 within reaction vessel 201 such that rotation of shaft 206 will cause material within the reaction vessel to be intimately mixed and progressed through the reaction vessel. An outlet pipe 209 at the end of reaction vessel 201 remote from inlet 203 leads to a two-way valve 210. From valve 210 a pipe 211 leads to a conventional animal feed compounding plant (not shown). A second pipe 212 leads via pump 213 to a jacketed storage vessel 214, from which material can be drawn off via valve 215 and pipe 215 as required. Storage vessel 214 is provided with stirring and heating means (not shown) to maintain contents in a flowable state if necessary. If desired, reaction vessel 201 can be inclined downwards at a slight angle to the horizontal to assist flow of material.

Example 2

Pilot-scale equipment as just described with reference to Figure 2 was used to prepare enzyme-treated tapioca meal on a continuous basis as follows. The reaction chamber had a length of 1200 mm and an internal diameter of 120 mm.

Tapioca meal that had been milled to a particle size of less than 300 microns was fed "dry" (i.e. at its natural moisture content of about 12% by weight) into the apparatus of Figure 2 via inlet 203. An aqueous slurry containing 0/1% by weight of commercially-available alpha-amylase enzyme "TERMAMYL 120L", was fed in via inlet 204, at a level of 12% slurry by weight of the dry meal. Steam was fed in via inlet 205, adding a further 6% moisture and raising the temperature in the reactor at steady state to about 100°C. The tapioca meal remained in the reactor for about one minute emerging as a highly viscous dark liquid. Throughput was about 40 kg per hour. This material was added at various levels to a compound cattle feed and pelleted. Even at an inclusion level of only 1% by weight, it lead to a noticeable increase in pellet hardness.

Example 3

Example 2 was repeated using "dry" potato starch instead of tapioca meal. This had a natural moisture content of about 10% by weight. All other reaction conditions were as in Example 2. The product emerged from the reactor as a highly viscous dark liquid that set to a hard solid at ambient temperature.

Example 4

The animal feed pellet binding properties of the partially-hydrolysed starch material is demonstrated by the following example.

Two compound cattle feeds were pelleted using a small ring-roll press, and the pellets allowed to cool. The first formulation (A) contained 4% by weight of hydrolysed tapioca meal prepared at 60% solids using the procedure of Example 2. The second formulation (B) was identical except that the 4% hydrolysed tapioca meal was replaced by 3% natural tapioca meal (12% moisture) plus 1% added water, leading to an identical noisture content in the overall formulaton. The friability of the pelleted feeds were compared using a standardised test method, involving abrading a sample of each feed in a food blender. The results, in terms of the fines (ml) per 1000 ml of pelleted product, are set out in Table 1 for two press throughputs.

## Table 1

| Throughput | Formulation | Relative Friability |
|---|---|---|
| 80 kg/hr | A | 22 |
| 80 kg/hr | B | 58 |
| 60 kg/hr | A | 21 |
| 60 kg/hr | B | 50 |

The lower friability due to the inclusion of the hydrolysed tapioca was significant in product performance terms.

## Example 5

This example compared the pellet binding properties of the hydrolysed starch material of the invention and molasses.

Two compound cattle feeds, each containing 2% added fat and 5% binder, by weight, were pelleted using a small ring-roll press, and allowed to cool. The first formulation (C) contained as binder the partially hydrolysed tapioca meal prepared at 65% solids using the procedure of Example 1. The second formulation (D) contained molasses as binder. The pelleted products were subjected to the same friability test used in Example 4, and the results are set out in Table 2 for two press throughputs.

## Table 2

| Throughput | Formulation | Relative Friability |
|---|---|---|
| 80 kg/hr | C | 35 |
| 80 kg/hr | D | 49 |
| 60 kg/hr | C | 24 |
| 60 kg/hr | D | 39 |

These results show that molasses had significantly inferior pellet binding properties.

### Example 6

This example demonstrates the potential use of the partially-hydrolysed starch material of the invention as a means for enabling a higher level of fat to be included in a pelleted feed.

A control formulation comprising a standard cattle feed containing 3% added fat and 6% molasses binder, by weight, was pelleted at 60 kg/hr using a small ring-roll press.

By comparing friability test results, obtained using the test described in Example 4, it was found that in a range of pelleted cattle feeds of the same basic formulation containing 6% hydrolysed tapioca as binder, an additional 1.3% by weight fat could be included without the physical properties of the pellets becoming inferior. The test feeds were all pelleted using the same press as in the control experiment, at the same throughput. The hydrolysed tapioca was prepared by the method of Example 1, at 65% solids.

Example 7

This example shows the improvement in feed pellet hardness that can be obtained by using the hydrolysed starch material of the invention as a pellet binder instead of molasses.

A standard compound cattle feed was used as the base material, in four samples. Hydrolysed tapioca meal, prepared as per Example 2 at 65% solids, was added to samples E and G at a level of 4% and 8% by weight respectively. Control samples F and H contained 4% and 8% by weight molasses. Each sample was pelleted using a small ring-roll press. The pellets were allowed to "cure" for 48 hours, and then the hardness was assessed using the standard Kahl hardness test. The results are given in Table 3.

Table 3

| Formulation | Binder | Kahl hardness (kg) |
|---|---|---|
| E | 4% hydrolysed tapioca | 4.5 |
| F | 4% molasses | 3.5 |
| G | 8% hydrolysed tapioca | 4.5 |
| H | 8% molasses | 2.8 |

These results show that the binder of the invention resulted in a significant harder pellet. Whereas increasing the inclusion level of the hydrolysed tapioca left the hardness constant at a high figure, increasing the molasses lead to a drop in pellet hardness.

The beneficial effects described in Examples 4 to 7 are also obtained in the manufacture of pelleted feeds using large commercial-scale presses operating at high throughputs.

CLAIMS

1.    A process for the manufacture of a starch-rich material useful as an animal feedstuff ingredient, in which process a starch-rich meal is subjected to hydrolysis by an alpha-amylase enzyme capable of hydrolysing starch to dextrins having a degree of polymerisation (DP) in the range 4 to 10, the hydrolysis being conducted at a total dry solids content of at least 50% by weight.

2.    A process according to claim 1, conducted at a total dry solids content of at least 60% by weight.

3.    A process according to claim 1, conducted at a total dry solids content of at least 70% by weight.

4.    A process according to any one of claims 1 to 3, wherein the starch-rich meal has a maximum particle size of less than 1000 microns.

5.    A process according to any one of claims 1 to 4, wherein the enzyme is a heat-stable endo-amylase which will hydrolyse 1,4-alpha-glucosidic linkages in amylose and amylopectin at random.

6.    A process according to claim 5, wherein the enzyme hydrolyses starch to dextrins of DP 5.

7.    A process according to any one of the preceding claims conducted at a temperature in the range 80 to 110°C.

- 18 -                Q.1087 GB
                        **0120573**

8.  A process according to any one of the preceding claims conducted on a continuous basis in which the starch-rich meal and the enzyme are mixed and passed through a heated reaction chamber.

9.  A process according to any one of the preceding claims in which the starch hydrolysis is conducted during a processing time of less than 10 minutes.

10. A process according to claim 1, in which a mixture of tapioca meal having a maximum particle size of less than 1000 microns, moisture and a heat-stable endo-amylase enzyme capable of hydrolysing starch to dextrins having a DP in the range of 4 to 10, the mixture having total solids content of at least 60% by weight, is heated to a temperature in the range 80 to 110°C.

11. A process according to any one of the preceding claims, in which the hydrolysed starch-rich meal dried.

12. A process according to claim 11, in which the hydrolysed starch-rich material is blended with a carrier material and then dried.

13. A process for the preparation of a compound animal feedstuff in the form of pellets or similar compressed particles, in which process a hydrolysed starch-rich material produced by the process of any one of the preceding claims is blended with a compound feed formulation which is then formed into pellets or similar compressed particles.

14. The use of a starch-rich material prepared by a process according to any one of claims 1 to 12, as a conditioning agent for non-hydrolysed starch-rich material in pellet form.

15. A method for improving the handling and properties of natural starch-rich meal, in which the natural starch-rich meal is blended with a minor proportion of a starch-rich meal that has been subjected to enzyme hydrolysis by a process according to any one of claims 1 to 12, and the blend of natural and hydrolysed starch-rich material is pelleted.

16. A method according to claim 15, in which natural tapioca meal is blended with a minor proportion of partially hydrolysed tapioca meal and the blend is pelleted.

17. A process according to claim 1, substantially as hereinbefore described with reference to either of the accompanying drawings.

18. Natural tapioca meal in the form of pellets, containing a minor proportion of tapioca meal that has been subjected to enzyme hydrolysis by a process according to any one of claims 1 to 12.

Fig.1.

Fig.2.